# EUROPEAN PATENT APPLICATION

(11) **EP 2 517 686 A2**
(43) Date of publication of application: **31.10.2012**
(21) Application number: 12165739.9
(22) Date of filing: 26.04.2012
(51) Int. Cl.: A61J 1/10

(54) **A kit comprising a multi-chamber container**

(30) Priority: 29.04.2011 ES 201130683
(71) Applicant: Combino Pharm, S.L., 08970 Saint Joan Despi (ES)
(72) Inventor: Abascal Martinez, Natalia, 08011 Barcelona (ES); Sicart Abello, Noemi, 43204 Reus (Tarragona) (ES); Puigvert Colomer, Marina, 08014 Barcelona (ES)
(74) Representative: ZBM Patents

(57) **Abstract**

The present invention refers to a kit comprising a multi-chamber container characterised in that the multi-chamber container comprises at least one chamber A and one chamber B, wherein the chamber A contains a pharmaceutically acceptable solution of at least one active ingredient and the chamber B contains a pharmaceutically acceptable solution for reconstitution of the solution contained in the chamber A. The invention provides a new kit for itraconazole for parenteral administration that consists of a double-chamber biocompatible plastic bag that comprises one chamber A containing a solution of itraconazole and one chamber B containing a pharmaceutically acceptable solution for the reconstitution of the itraconazole solution contained in the chamber A.

## Description

The present invention refers to a kit comprising a multi-chamber container characterised in that the multi-chamber container comprises at least one chamber A and at least one chamber B, wherein the chamber A contains a pharmaceutically acceptable solution of at least one active ingredient and the chamber B contains a pharmaceutically acceptable solution for the reconstitution of the solution contained in the chamber A.

### BACKGROUND OF THE INVENTION

Many active ingredients used in pharmacological applications are poorly soluble in water. Such active ingredients may commonly be formulated as a suspension, in which case the lack of solubility of the active ingredient in water does not normally have a negative impact on its bioavailability. However, in situations where a solution of the active ingredient is required, the lack of solubility of the active ingredient in water is a significant barrier to achieving the desired concentration of the active ingredient.

Thus, for example, the development of pharmaceutically effective azole-based antifungal active ingredient compositions, such as itraconazole, fluconazole, voriconazole, posaconazole, clotrimazole, ketoconazole, clotrimazole, econazole, saperconazole, ravuconazole, isavuconazole, miconazole and tioconazole, is severely hindered by the fact that these antifungal agents are poorly soluble in water.

As described in documents EP0149197 and EP0335545, the solubility and bioavailability of these poorly water-soluble active ingredients can be increased by complexation with cyclodextrins or derivatives thereof. Itraconazole is a broad-spectrum antifungal agent developed for oral, parenteral and topical use that was described for the first time in document EP0006711.

Itraconazole is defined as (±)-1-*sec*-butyl-4-[*p*-[4-[p-[[(2*R**,4*S**)-2-(2,4-dichlorophenyl)-2- (1*H*-1,2,4-triazol-1-ylmethyl)-1,3-dioxolan-4-yl]methoxy]phenyl]-1-piperazinyl]phenyl]-Δ²-1,2,4-triazolin-5-one, or, alternatively, as 4-[4-[4-[4-[[2-(2,4-dichlorophenyl)-2-(1*H*-1,2,4-triazol-1-ylmethyl)-1,3-dioxolan-4-yl]methoxy]phenyl]-1-piperazinyl]phenyl]-2,4-dihydro-2-(1-methylpropyl)-3*H*-1,2,4-triazol-3-one. Itraconazole contains three asymmetric carbon centres: one in the *sec*-butyl side chain of the triazolone and two in the dioxolane ring. As a result, itraconazole has eight possible stereoisomers: (*R, R, R*), (*S, S, S*), (*R, R, S*), (*S, S, R*), (*R, S, S*), (*R, S, R*), (*S, R, S*) and (*S, R, R*).

The preferred compound of itraconazole is the form (±)*cis*-itraconazole, which comprises a 1:1:1:1 mixture of the (*R, S, S*), (*R, S, R*), (*S, R, S*) and (*S, R, R*) isomers.

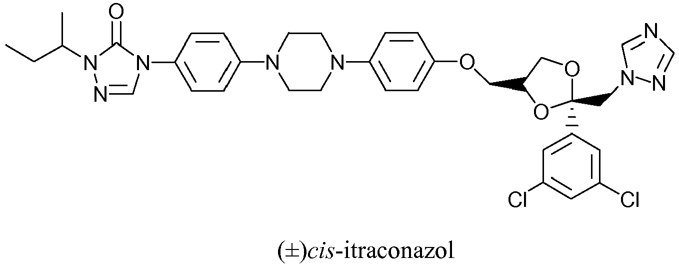

The term "itraconazole", as used hereinafter, should be interpreted as referring to (±)*cis*-itraconazole and includes both the free-base form and pharmaceutically acceptable addition salts thereof. Acid addition salts of itraconazole can be obtained by treating the free-base form with the appropriate acid. Appropriate acids include, for example, inorganic acids such as hydrochloric acid, hydrobromic acid, sulfuric acid, nitric acid, phosphoric acid and analogues thereof; or organic acids such as, for example, acetic acid, propanoic acid, hydroxyacetic acid, 2-hydroxy propanoic acid, 2-oxopropanoic acid, ethanedioic acid, propanedioic acid, butanedioic acid, (*Z*)-butenedioic acid, (*E*)-butenedioic acid, 2-hydroxybutanedioic acid, 2,3-dihydroxybutanedioic acid, 2-hydroxy-1,2,3-propanetricarboxylic acid, methanesulfonic acid, ethanesulfonic acid, benzenesulfonic acid, 4-methylbenzenesulfonic acid, cyclohexanesulfamic acid, 2-hydroxybenzoic acid, 4-amino-2-hydroxybenzoic acid and analogues thereof.

Itraconazole is currently marketed under the name SPORANOX in the form of oral capsules, oral solution and concentrate for solution for infusion.

In particular, the SPORANOX parenteral administration kit containing itraconazole includes (see the summary of product characteristics (SPC) for SPORANOX):
- A 75 mL flexible polypropylene infusion bag containing about 50 mL of 0.9% (weight/volume) NaCl injectable solution.
- A type I siliconised glass ampoule containing 25 mL of a 10mg/mL aqueous itraconazole concentrate, wherein the itraconazol is in the form of trihydrochloride salt, which is formed *in situ.*
- An extension line formed by a polyvinyl chloride tube with a two-way shut-off valve and an in-line filter.

The SPORANOX kit does not include the infusion line with drip chamber.

As indicated in section 6.6, special precautions for disposal and other handling of the SPC for SPORANOX, the itraconazole concentrate should be removed from the glass ampoule using a needle of appropriate length, which is not included in the SPORANOX kit, and subsequently the entire volume of the itraconazole concentrate should be added to the infusion bag by puncturing the port and gently mixing the contents of the bag once all the concentrate has been added. This infusion mixture should be used immediately; if not, the storage time should not exceed 24 h at a temperature of 2-8 °C.

The infusion mixture must then be incorporated into the infusion line with drip chamber, which is not included in the SPORANOX kit, and this, in turn, must be connected to the extension line with two-way shut-off valve and the in-line filter, included in the SPORANOX kit. Finally, the flow-control device is opened until all the air has been expelled from the infusion line and extension line so that the infusion is ready for intravenous infusion into the patient. As indicated in the SPC for SPORANOX, infusion is stopped once 60 mL, equivalent to 200 mg of itraconazole, has been infused.

The SPORANOX kit presents several drawbacks, mainly as a result of the high degree of handling required to prepare the infusion mixture before it can be administered to the patient and the tendency of itraconazole to precipitate in water due to its low solubility.

Thus, the 10 mg/mL itraconazole concentrate in SPORANOX is a viscous solution with a density of 1.10-1.15 g/mL, thus making its complete removal from the glass ampoule with a syringe an arduous task that inevitably results in loss of material, thus resulting in variations in the actual dose subsequently administered to the patient. Furthermore, removal of the concentrate using a syringe is not a sterile operation, thus meaning that the risk of contamination during this procedure is real, high and of great significance for a product that is to be administered intravenously.

Likewise, introduction of the SPORANOX concentrate into the infusion bag using a syringe is a slow and tedious process as the incorporation of excess air should be avoided. The incorporation of excess air can result in foaming of the dilute solution, thus making homogenisation of the solution more difficult and therefore delaying administration. Furthermore, the inventors of the present invention have observed visible turbidity during introduction of the SPORANOX concentrate into the infusion bag using a syringe due to the transient precipitation of itraconazole, which afterwards redissolves. Indeed, the fact that the commercial pharmaceutical SPORANOX kit contains an in-line filter clearly suggests the danger that itraconazole may precipitate at any time or that not all the itraconazole remains completely dissolved after reconstitution. Furthermore, the SPC for SPORANOX notes that itraconazole may precipitate when the 25 mL of concentrate are diluted in solutions for infusion other than the 50 mL of 0.9% NaCl solution for infusion (weight/volume), thus highlighting the limited versatility of this pharmaceutical as far as choice of reconstitution solution is concerned.

Another drawback of the SPORANOX kit is that only 200 mg of the 250 mg of itraconazole contained in the concentrate is administered to the patient, thus meaning that 50 mg of itraconazole, in other words 20% of total itraconazole, is directly discarded and therefore wasted. Section 2.2, posology and method of administration, of the SPC for SPORANOX indicates that two infusions should be performed per day for the first two days of treatment, followed by one infusion per day thereafter. This implies a total of 16 infusions in the maximum 14 days over which SPORANOX can be administered, thus resulting in the waste of a total of 800 mg of itraconazole over the treatment period.

There is therefore a need to provide alternative active ingredient kits, preferably for poorly water-soluble active ingredients, such as itraconazole, which must be reconstituted prior to parenteral administration that do not require such extensive handling during preparation of the reconstituted solution, thereby reducing the risk of contamination and avoiding the numerous drawbacks referred to above.

### DESCRIPTION OF THE INVENTION

The present invention provides an alternative kit that allows to mix a solution containing a active ingredient with a solution for the reconstitution of the solution containing the active ingredient in order to obtain the final reconstituted solution to be administered to the patient.

The kit of the present invention provides the reconstituted solution for parenteral administration in a faster and more efficient manner without the need to handle the solution containing the active ingredient. The risk of contamination is therefore prevented and a constant dose for the patient ensured.

On the other hand, and surprisingly, the authors of the present invention have observed that, when using the kit of the present invention, much less precipitation of the active ingredient is observed when mixing the solution containing the active ingredient, preferably a poorly water-soluble active ingredient, with the solution for reconstitution of the solution containing the active ingredient than if the solution containing the active ingredient is injected into the solution for the reconstitution of the active ingredient solution, which is the standard practice for preparing reconstituted solutions for parenteral administration. The kit of the present invention therefore reduces the risk of precipitation of the active ingredient, preferably a poorly water-soluble active ingredient, in reconstituted solutions that are administered to patients parenterally.

In its first aspect, the present invention refers to a kit comprising a multi-chamber container characterised in that the multi-chamber container comprises at least one chamber A and at least one chamber B, wherein the chamber A contains a pharmaceutically acceptable solution of at least one active ingredient and the chamber B contains a pharmaceutically acceptable solution for the reconstitution of the solution contained in the chamber A.

The multi-chamber container of the present invention comprises, for example, multi-chamber vials, multi-chamber ampoules, multi-chamber syringes, multi-chamber bottles and multi-chamber bags, preferably multi-chamber bags. The multi-chamber container of the present invention may comprise from 2 to 5 chambers. Preferably the multi-chamber container of the present invention comprises from 2 to 3 chambers such that at least one chamber contains a pharmaceutically acceptable solution of at least one active ingredient and at least another chamber contains a pharmaceutically acceptable solution for the reconstituion of the active ingredient solution.

The multi-chamber container of the present invention may present different configurations. For example, the chambers may lie adjacent to each other or, alternatively, they may be contained one within another in a concentric or non-concentric manner.

In a preferred embodiment of the present invention, the multi-chamber container is a container comprising one chamber A and one chamber B, wherein the chamber A contains a pharmaceutically acceptable solution of at least one active ingredient and the chamber B contains a pharmaceutically acceptable solution for the reconstitution of the solution contained in the chamber A, so that the container contains the means for mixing the solution contained in the chamber A and the solution contained in the chamber B inside the same multi-chamber container by means of total or partial rupture of the barrier between the chamber A and the chamber B.

The chambers of the multi-chamber container of the present invention, especially when the multi-chamber container is a multi-chamber bag, may be separated by a weld, in other words by a welded separation zone, by a separation zone made from any breakable plastic material or by a continuous closure line in which a break valve is inserted.

Alternatively, the multi-chamber container of the present invention is a container comprising a chamber A containing a pharmaceutically acceptable solution of at least one active ingredient, a chamber B containing a pharmaceutically acceptable solution for the reconstitution of the solution contained in the chamber A and a chamber C which is empty and in which mixing of the solution contained in the chamber A with the solution contained in the chamber B may be performed. Optionally, the multi-chamber container of the present invention also comprises a container comprising a chamber A containing a pharmaceutically acceptable solution of at least one active ingredient, a chamber B containing a pharmaceutically acceptable solution of at least one active ingredient and a chamber C containing a pharmaceutically acceptable solution for the reconstitution of the solutions contained in the chambers A and B.

In another preferred embodiment of the present invention, the multi-chamber container is a multi-chamber bag made from biocompatible plastic. The multi-chamber biocompatible plastic bag of the present invention is preferably a double-chamber biocompatible plastic bag.

Chambers A and B of the multi-chamber biocompatible plastic bag of the present invention may be separated by a weld, in other words by a welded separation zone, by a separation zone made from any breakable plastic material or by a continuous closure line in which a break valve is inserted. Chambers A and B of the multi-chamber biocompatible plastic bag of the present invention are preferably separated by a weld.

The weld separating chambers A and B of the multi-chamber biocompatible plastic bag of the present invention preferably consist of a single weld line that may be oblique or, preferably, vertical. The weld that separates chambers A and B of the multi-chamber biocompatible plastic bag of the present invention may optionally consist of more than one weld line, preferably consisting of two weld lines that may be in the form of an "L" or a "V".

Chambers A and B of the multi-chamber biocompatible plastic bag of the present invention are preferably each connected to the exterior by a port through which each of the solutions contained in each of chambers A and B may be introduced, and the solution formed upon mixing the two solutions initially contained in each of chambers A and B may be removed. Alternatively, the multi-chamber biocompatible plastic bag of the present invention may also include a third port through which the solution formed upon mixing the two solutions initially contained in each of chambers A and B can be removed.

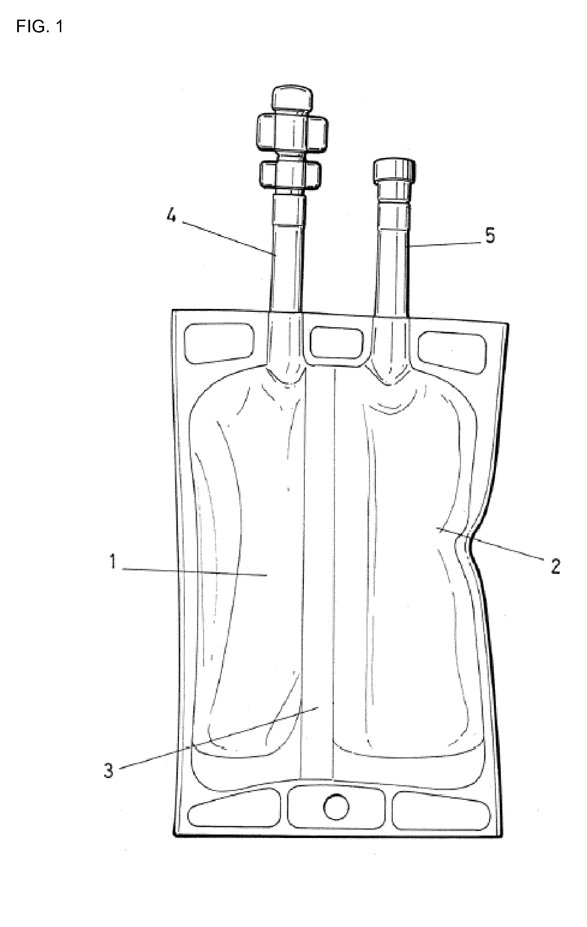

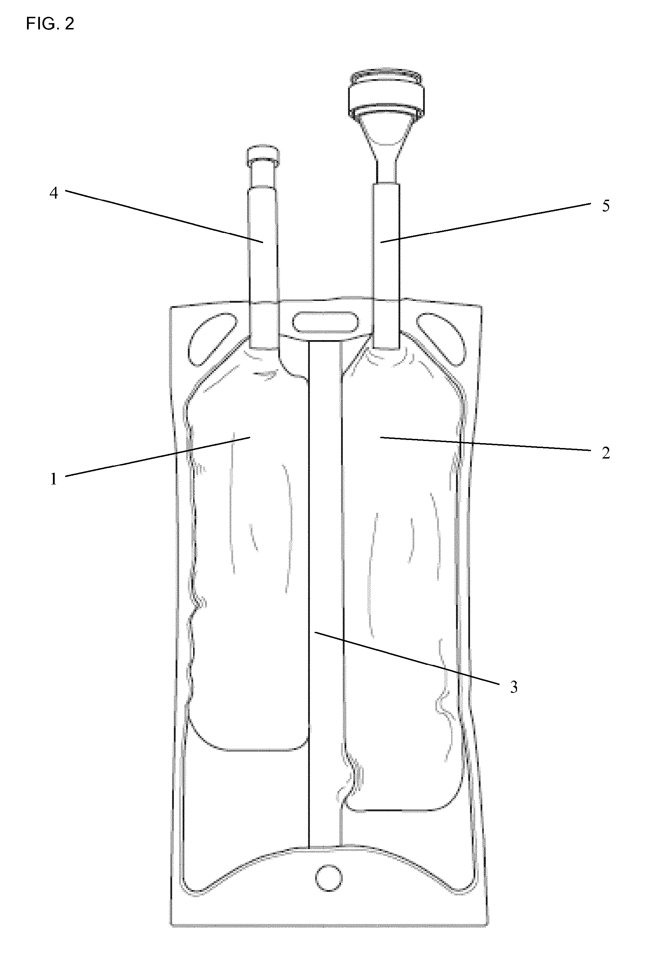

Figure 1 and Figure 2 show preferred embodiments of the multi-chamber biocompatible plastic bag of the present invention:
1. Chamber A.
2. Chamber B.
3. Vertical weld line.
4 and 5. Ports to the exterior.

Figure 2 shows a preferred embodiment of the multi-chamber biocompatible plastic bag of the present invention in which the dessign of the biocompatible plastic bag has been improved in order to facilitate the filling of the chamber A with about 25 ml of the pharmaceutically acceptable solution of at least one active ingredient and the filling of the chamber B with about 50 ml of a pharmaceutically acceptable solution for the reconstitution of the solution contained in the chamber A.

Prior to parenteral administration in the patient, the pharmaceutically acceptable solutions contained in chamber A and chamber B of the multi-chamber biocompatible plastic bag of the present invention are mixed by totally or partially breaking the weld that initially separates chambers A and B. This breakage preferably occurs by gently manually pressing at least one of the two chambers A or B. More preferably, the breakage occurs upon folding the bag perpendicular to the separation between the two chambers A and B and manually pressing the two chambers A and B. Optionally, it is also possible to equip the outer wall of the separation zone between the two chambers A and B with a breakage tab, preferably two breakage tabs. Chambers A and B of the multi-chamber biocompatible plastic bag of the present invention may have the same or different volumes, preferably chambers A and B have different volumes. The volumes of each chamber A and B may be comprised of from 5 mL to 200 mL, preferably comprised of from 10 mL to 100 mL, and more preferably comprised of from 20 mL to 60 mL. Even more preferably, Chamber A may have a volume comprised of from 20 mL to 30 mL, most preferably from 25 to 27 mL, and chamber B may have a volume comprised of from 40 to 60 mL, most preferably comprised of from 50 to 52 mL.

The multi-chamber biocompatible plastic bag of the present invention consists of a polymeric material that is preferably presented in the form of multi-layer films. In one referred embodiment, the multilayer film consists of a film formed from two to seven layers, preferably five layers, although a single film of polymeric material may be used for the multi-chamber biocompatible plastic bags of the present invention. The polymeric materials of the multilayer films should be chosen so as to ensure a weld that is both effective at separating the solutions contained in each of the two chambers A and B and can easily be broken in order to mix the two solutions when required.

Examples of the polymer materials from which the multi-chamber biocompatible plastic bag of the present invention may be made include, but are not limited to, polyolefins, cyclic polyolefins, polyesters and polyamides, preferably polyolefins. These polymeric materials may be used alone, or two or more classes may be mixed and used.

The polyolefins used preferably comprise homopolyethylene, a polyethylene-olefin copolymer, a polypropylene homopolymer, a polypropylene-olefin copolymer, etc.

In a preferred embodiment of the present invention the multi-chamber biocompatible plastic bag may be made by a mixture of modified ethylenepropylene copolymer, styrene-based elastomer and polyethylene.

The polymeric materials from which the multi-chamber biocompatible plastic bag of the present invention is made may optionally contain a material that has a barrier effect towards gases (such as oxygen, carbon dioxide or water vapour) in order to prevent the diffusion of these gases both into and out of the bag. Examples of materials with a barrier effect towards gases include, but are not limited to, polyvinyl alcohol (PVA), ethylene-vinyl alcohol copolymer (EVOH), polyvinyl acetate (PVAC), ethylene-vinyl acetate copolymer (EVA), polyvinylidene chloride (PVDC), polyglycolic acid, ethylcellulose, cellulose acetate, nitrocellulose, high density polyethylene (HDPE), medium density polyethylene (MDPE), nylon, polystyrene (PS), polycarbonate (PC), polyacrylonitrile, etc.

The polymeric materials from which the multi-chamber biocompatible plastic bag of the present invention is made may optionally contain a material that has a barrier effect towards light, which is useful in the event that at least one of the active ingredients contained is light-sensitive, such as itraconazole for example.

The multi-chamber biocompatible plastic bag of the present invention is preferably placed inside a blister to prevent the diffusion of gases and/or to protect the active ingredient or active ingredients contained within from light. The blisters that cover the multi-chamber biocompatible plastic bag of the present invention are preferably plastic bags consisting of one or more layers, preferably two or three layers. Examples of the plastics from which the blister used in the present invention is made include, but are not limited to, polyester, polypropylene and polyethylene. The blister used in the present invention is preferably a double-layered film of polyester/polyethylene or a triple-layered film of polyester/polyethylene/polypropylene.

The thickness of the plastic from which the blister is made is directly related to its efficacy in terms of preventing gas diffusion. The blister used in the present invention has a thickness comprised of from 40 µm to 180 µm, preferably comprised of from 100 µm to 150 µm, and even more preferably about 112 µm.

Optionally, the blister used in the present invention may optionally be a metallised blister to protect the active ingredient or active ingredients contained therein from light.

In a preferred embodiment of the present invention, the pharmaceutically acceptable solution contained in the chamber A contains a pharmaceutically acceptable solution, preferably a pharmaceutically acceptable aqueous solution, of at least one poorly water-soluble active ingredient, preferably an antifungal agent wherein the antifungal agent is an azole derivative, more preferably itraconazole, fluconazole, voriconazole, posaconazole, clotrimazole, ketoconazole, econazole, saperconazole, ravuconazole, isavuconazole, miconazole or tioconazole, even more preferably itraconazole. In a preferred embodiment of the present invention, the pharmaceutically acceptable solution, preferably pharmaceutically acceptable aqueous solution, contained in the chamber A comprises a single active ingredient, preferably itraconazole.

In another preferred embodiment of the present invention, the pharmaceutically acceptable solution contained in the chamber A is a pharmaceutically acceptable aqueous solution of the azole derivative, preferably itraconazole, with a concentration comprised of from 1 mg/mL to 30 mg/mL, preferably comprised of from 5 mg/mL to 20 mg/mL. More preferably, the pharmaceutically acceptable solution contained in the chamber A is a pharmaceutically acceptable aqueous solution of itraconazole with a concentration about 10 mg/mL.

The pharmaceutically acceptable aqueous solution of the azole derivative, preferably itraconazole, of the present invention has a volume comprised of from 5 mL to 100 mL, preferably comprised of from 10 mL to 50 mL, more preferably comprised of from 20 mL to 30 mL, and even more preferably about 25 mL.

In a further preferred embodiment of the present invention, the pharmaceutically acceptable solution contained in the chamber A comprises at least one inclusion complex of the azole derivative-cyclodextrin, preferably itraconazole-cyclodextrin. Non-limiting examples of cyclodextrins of the present invention are α-, β- or γ-cyclodextrins optionally substituted by hydroxyethyl, hydroxypropyl, dihydroxypropyl, methylhydroxyethyl, methylhydroxypropyl, ethyl hyd roxyethyl, ethylhydroxypropyl, glucosyl, maltosyl, maltotriosyl or sulfoalkly ether groups, such as sulfobutyl ether for example. The preferred cyclodextrin used in the present invention is hydroxypropyl-β-cyclodextrin.

The ratio between the weight of cyclodextrin and the weight of the azole derivative, preferably itraconazole, in the pharmaceutically acceptable solution contained in the chamber A of the present invention is comprised of from 1:1 to 100:1, preferably comprised of from 20:1 to 50:1, and more preferably about 40:1. In a preferred embodiment of the present invention, the pharmaceutically acceptable solution of the azole derivative, preferably itraconazole, has a ratio between the weight of hydroxypropyl-β-cyclodextrin and the weight of the azole derivative, preferably itraconazole, of about 40:1. In another preferred embodiment of the present invention, the pharmaceutically acceptable solution contained in the chamber A further comprises a polymeric solubiliser or a mixture thereof. Non-limiting examples of polymeric solubilisers of the present invention are polyethylene-propylene glycol copolymers, polyoxyethylene-polyoxypropylene copolymers (poloxamer 188, poloxamer 407, poloxamer 124 or poloxamer 184) or hydroxy(polyoxyethylene)-polyoxypropylene-polyoxyethylene copolymers. The preferred polymeric solubiliser used in the present invention is a copolymer of polyoxyethylene and polyoxypropylene, more preferred poloxamer 188. Optionally, the pharmaceutically acceptable solution contained in the chamber A of the present invention may simultaneously contain an inclusion complex of the azole derivative-cyclodextrin, preferably itraconazole-cyclodextrin and also a polymeric solubiliser or a mixture thereof.

In a further preferred embodiment of the present invention, the pharmaceutically acceptable solution contained in the chamber A further comprises one or more co-solvents, preferably selected from the group consisting of ethanol, propylene glycol, oils or glycerine, more preferably propylene glycol.

The ratio between the weight of the co-solvent and the weight of the azole derivative, preferably itraconazole, in the pharmaceutically acceptable solution contained in the chamber A of the present invention is comprised of from 1:5 to 10:1, preferably comprised of from 1:1 to 5:1, more preferably comprised of from 2:1 to 3.5:1, and most preferably about 2.6:1. In a preferred embodiment of the present invention, the pharmaceutically acceptable solution of the azole derivative, preferably itraconazole, has a ratio between the weight of propylene glycol and the weight of the azole derivative, preferably itraconazole, of about 2.6:1.

In another preferred embodiment of the present invention, the pharmaceutically acceptable solution contained in the chamber A further comprises one or more pharmaceutically acceptable acids, so that the poorly water-soluble active ingredient, preferably an antifungal agent wherein the antifungal agent is an azole derivative, more preferably itraconazole, is in the form of a salt. Pharmaceutically acceptable acids comprise, for example, inorganic acids such as hydrochloric acid, hydrobromic acid, sulfuric acid, nitric acid, phosphoric acid and analogues thereof; or organic acids such as, for example, acetic acid, propanoic acid, hydroxyacetic acid, 2-hydroxy propanoic acid, 2-oxopropanoic acid, ethanedioic acid, propanedioic acid, butanedioic acid, (*Z*)-butenedioic acid, (*E*)-butenedioic acid, 2-hydroxybutanedioic acid, 2,3-dihydroxybutanedioic acid, 2-hydroxy-1,2,3-propanetricarboxylic acid, methanesulfonic acid, ethanesulfonic acid, benzenesulfonic acid, 4-methylbenzenesulfonic acid, cyclohexanesulfamic acid, 2-hydroxybenzoic acid, 4-amino-2-hydroxybenzoic acid and analogues thereof, preferably hydrochloric acid.

In a preferred embodiment of the present invention, the pharmaceutically acceptable solution contained in the chamber A is an aqueous solution containing an inclusion complex of itraconazole and hydroxypropyl-β-cyclodextrin, propylene glycol and hydrochloric acid, so that the itraconazole is preferably in the form of a salt, preferably in the form of a trihydrochloride. The solution contained in the chamber B of the present invention comprises a pharmaceutically acceptable solution for the reconstitution of the pharmaceutically acceptable solution contained in the chamber A. A solution for the reconstitution of the solution contained in the chamber A is a solution containing the components required to ensure that, once the contents of the chambers A and B have been combined, the resulting solution can be used directly for the intended purpose, such as intravenous infusion for example. Non-limiting examples of the solution contained in chamber B are a NaCl solution, a glucose solution, a dextrose solution or a mixture thereof, preferably an about 0.9% NaCl solution (weight/volume).

The pharmaceutically acceptable solution contained in the chamber B of the present invention has a volume comprised of from 10 mL to 200 mL, preferably comprised of from 20 mL to 100 mL, more preferably comprised of from 40 mL to 60 mL. Even more preferably, the pharmaceutically acceptable solution contained in the chamber B of the present invention has a volume about 50 mL.

In a second aspect, the present invention provides a kit comprising a double-chamber biocompatible plastic bag comprising one chamber A and one chamber B, characterised in that the chamber A contains a volume comprised of from 20 mL to 30 mL of a pharmaceutically acceptable aqueous solution of itraconazole with a concentration about 10 mg/mL and the chamber B contains a volumen comprised of from 40 mL to 60 mL of a pharmaceutically acceptable NaCl solution with a concentration about 0.9% (weight/volume).

In a preferred embodiment of the present invention, the pharmaceutically acceptable aqueous solution of itraconazol with a concentration about 10 mg/ml contained in the chamber A further comprises hydroxypropyl-β-cyclodextrin, propylene glycol and hydrochloric acid.

In another preferred embodiment of the present invention, the chamber A contains about 25 mL of a pharmaceutically acceptable aqueous solution of itraconazole with a concentration about 10 mg/mL and also, preferably, hydroxypropyl-β-cyclodextrin, propylene glycol and hydrochloric acid, and the chamber B contains about 50 mL of a pharmaceutically acceptable NaCl solution with a concentration about 0.9% (weight/volume).

In another preferred embodiment, the kit of the present invention further comprises a blister, preferably metallised, which covers the multi-chamber container and an in-line filter and, optionally, an extension line consisting of a plastic tube with a two-way shut-off valve.

The kit of the present invention may be used as a medicinal product.

The pharmaceutical kit of the present invention, which preferably contains an antifungal agent, where the antifungal agent is an azole derivative, preferably itraconazole, may be used for the treatment of histoplasmosis, aspergillosis, candidiasis and cryptococcosis.

The process for preparing the kit of the present invention comprises, for example, the following steps:
1) Preparation of the pharmaceutically acceptable active ingredient solution.
2) Preparation of the pharmaceutically acceptable solution for the reconstitution of the active ingredient solution.
3) Filtration of the pharmaceutically acceptable active ingredient solution and semi-automatic dosing into the chamber A of the multi-chamber container of the present invention.
4) Filtration of the pharmaceutically acceptable solution for the reconstitution of the active ingredient solution and semi-automatic dosing into the chamber B of the multi-chamber container of the present invention.
5) Sterilisation of the kit obtained in step 4).
6) Optionally, introduction of the kit obtained in step 5) into a blister.

Step 1 and the filtration process in step 3 can be undertaken either under an inert atmosphere, for example under nitrogen or argon, or without an inert atmosphere. Preferably, step 1 and the filtration process in step 3 are carried out under an inert atmosphere, preferably under nitrogen.

Steps 2, 4 and the dosing process in step 3 are preferably undertaken without an inert atmosphere.

Step 5, namely sterilisation of the kit obtained after dosing the corresponding solutions into chambers A and B, is preferably performed by steam sterilisation upon heating in an autoclave in order to achieve an F₀ equal or higher than 15. According to the European Pharmacopoeia, the parameter F₀ refers to the lethality of the equivalent time in minutes at a temperature of 121°C. The kit obtained after dosing the corresponding solutions into chambers A and B is preferably sterilised in an autoclave at a temperature of 121°C for 15 minutes, although other temperature and time conditions, for example 116°C for 50 minutes, 121°C for 45 minutes and 122.5°C for 15 min, may also be used.

Optionally, and preferably when at least one active ingredient, such as itraconazole, is photosensitive, the solution containing said active ingredient is maintained protected from light throughout the process indicated above. Optionally, the kit obtained after the sterilisation step is covered with a blister, which is preferably a metallised blister when at least one active ingredient, such as itraconazole, is photosensitive (step 6).

The kit of the present invention that comprises itraconazole presents numerous advantages with respect to the SPORANOX kit, which was first authorised for use in Europe by the UK Medicines Agency in 1996.

Thus, the kit of the present invention that comprises itraconazole does not require the manual handling of the itraconazole solution, which is very viscous at the preferred concentration of the present invention of about 10 mg/mL. The SPORANOX kit requires two manual handling steps for the itraconazole concentrate in order to prepare the solution that is subsequently administered parenterally to the patient: extraction of the itraconazole concentrate from the ampoule and its subsequent introduction into the infusion bag. These two steps are not required with the kit of the present invention, thus meaning that the solution to be administered to the patient is obtained in a faster and more efficient manner whilst eliminating the risk of contamination and loss of the active ingredient, thereby ensuring the administration of a constant dose to the patient.

The inventors of the present invention have established that manual extraction of the concentrate contained in the vial in the SPORANOX kit with a syringe results in the loss of about 3% of the starting concentrate. Furthermore, it should be noted that the losses that occur may vary, and may therefore be even higher, depending on the expertise with which this operation is performed. Use of the SPORANOX kit does not therefore ensure a constant dose for the patient.

As essentially no material is lost, other than the inevitable losses inherent to the use of an infusion line, the kit of the present invention comprising itraconazol allows to better adjust the amount of itraconazole that should be administered to the patient, namely 200 mg, without needing to squander the 20% of itraconazole wasted with the SPORANOX kit. This could result in a saving of 800 mg of itraconazole over the course of a 14-day treatment with this active ingredient.

Furthermore, and surprisingly, the kit of the present invention comprising itraconazol shows at least the same stability as the type I siliconised glass ampoule in which the itraconazol concentrate is contained in the SPORANOX kit. The inventors of the present invention have found that the stability of a solution of itraconazole, with the same qualitative and quantitative composition as the itraconazole concentrate contained in the SPORANOX kit, contained in the chamber A of the multi-chamber, preferably double-chamber biocompatible plastic bag, of the present invention is at least the same as that of the itraconazole concentrate contained in the type I siliconised glass ampoule of the SPORANOX kit even when the semi-automatic dosing of the itraconazole solution into the chamber A of the multi-chamber, preferably double-chamber biocompatible plastic bag, of the present invention is performed without an inert atmosphere. As such, a solution of itraconazole that is at least as stable as the itraconazole concentrate contained in the SPORANOX kit is obtained using a cheaper process that does not require the use of nitrogen or argon.

Moreover, according to the European Medicine Agency guidelines regarding the stability of new active ingredients and products (see CPMP/ICH/2736/99, dated on august 2003), the stability tests to which an active ingredient is subjected depend on the container. For example, for waterproof containers, such as glass, the sensitivity of the solution to external moisture or a potential loss of solvent are not relevant, so that the stability tests can be carried out under controlled conditions of humidity or under ambient air humidity. In contrast, in the case of aqueous solutions packaged in a semi-permeable containers, such as polyolefin bags, it has to evaluate the potential loss of solvents as the physical, chemical, biological and microbiological stability. Furthermore, these tests should be performed in very specific conditions. Therefore, the skilled person in the art would have expected that the stability of the itraconazole solution contained in the chamber A of the multi-chamber, preferably double-chamber biocompatible plastic bag, of the present invention, to be inferior to that of the same itraconazole solution contained in a glass ampoule given the fact that chemical inertness is not always satisfactory when liquid medicinal products come into contact with semi-permeable containers like plastic containers (see Tratado de Farmacia Galénica, Luzán 5, S.A. de Ediciones, 1993, Capítulo IV Estabilidad del medicamento pages 57 to 76). Likewise, the inventors of the present invention have found that, surprisingly, no changes occur to the multi-chamber, preferably double-chamber, biocompatible plastic bag containing a solution of itraconazole in chamber A even after high-temperature autoclave sterilisation, for example at 131°C or at 122.5°C. After this treatment, the skilled person in the art would have expected a hardening of the weld line that separates chambers A and B, which would make breaking the weld line in order to mix the solutions contained in chambers A and B more difficult or even impossible.

The authors of the present invention have observed that during the addition of the itraconazole concentrate of the SPORANOX kit into the infusion bag containing the 0.9% sodium chloride solution (weight/volume), a turbidity is formed due to the precipitation of the itraconazol. Once all the itraconazol concentrate is added into the infusion bag, the formed turbidity disappears after shaking the infusion bag for a period of time comprised of from two to three minutes. This precipitation of itraconazole in the SPORANOX kit occurs due to the fact that the equilibrium between itraconazole and hydroxypropyl-β-cyclodextrin established in the concentrate is displaced when the two solutions are mixed. The new system formed upon mixing these solutions therefore requires a certain time period, comprised of from two to three minutes, in order for the equilibrium between itraconazole and hydroxypropyl-β-cyclodextrin to be re-established and again form a transparent solution. The authors of the present invention have observed that, surprisingly, when mixing the itraconazole solution contained in the chamber A with the about 0.9% NaCl (weight/volume) solution contained in the chamber B of the kit of the present invention results in only a very slight, almost fain, turbidity that disappears within less than 15 seconds, with no need to shake the bag. In principle, the skilled person in the art would have expected that the precipitation of itraconazole in the kit of the present invention to be more abundant as the two solutions come into contact with each other much more abruptly and in a less sequential manner in comparision with the SPORANOX kit, where the concentrate is injected into the infusion bag via the port using a syringe, thus allowing more time for the system to re-establish the equilibrium between itraconazole and hydroxypropyl-β-cyclodextrin and redissolve any precipitate. The itraconazole-containing solution in the kit of the present invention therefore minimises the risk of itraconazole precipitation in the reconstituted solution that is to be administered to the patient.

As the risk of itraconazole precipitation is reduced, the risk that the optional in-line filter may be blocked is also minimised.

The term "kit" as used in the present invention refers to a set of products and utensils that are sufficient to achieve a certain goal, which can be marketed as a single unit. The kit of the present invention comprises the multi-chamber container comprising at least one chamber A containing a pharmaceutically acceptable solution of at least one active ingredient and at least one chamber B containing a pharmaceutically acceptable solution for the reconstitution of the solution contained in the chamber A, as well as this multi-chamber container optionally covered with a blister, preferably metallised, together with other utensils required for the parenteral administration of active ingredients. In a preferred embodiment of the present invention, the kit consists of a multi-chamber container comprising at least one chamber A containing a pharmaceutically acceptable solution of at least one active ingredient and at least one chamber B containing a pharmaceutically acceptable solution for the reconstitution of the solution in the chamber A.

Although the term "active ingredient" in the present invention includes both the active ingredient in its free-base form as well as in the form of a pharmaceutically acceptable addition salt, the term "poorly water-soluble active ingredient" as used in the present invention refers to the fact that the free-base form of the active ingredient requires preferably more than 1000 parts of water per 1 part of active ingredient in order to dissolved, and more preferably more than 10,000 parts of water per 1 part of active ingredient. The term "concentrate" is used in the present invention to refer to the solution of itraconazole with a concentration about 10 mg/mL from the SPORANOX kit. The term "biocompatible plastic" is used in the present invention to refer to any plastic that does not release any type of residue when in contact with the solutions of the present invention or, if it does release such residues, these are non-toxic and therefore compatible with parenteral administration.

The term "polymeric solubiliser" is used in the present invention to refer to any polymer that helps an active ingredient to be dissolved in water.

The term "under an inert atmosphere" as used in the present invention refers to maintaining the active ingredient solutions and/or the solutions for the reconstitution of the active ingredient solutions under an atmosphere consisting mainly of an inert gas, for example nitrogen or argon, and/or bubbling the active ingredient solutions and/or the solutions for the reconstitution of the active ingredient solutions with an inert gas, for example nitrogen or argon.

The term "about" when used in the present invention preceding a number and referring to it, is meant to designate any value which lies within the range defined by the number ±10% of its value, preferably a range defined by the number ±5%, more preferably a range defined by the number ±2%, still more preferably a range defined by the number ±1%. For example "about 10" should be construed as meaning within the range of 9 to 11, preferably within the range of 9.5 to 10.5, more preferably within the range of 9.8 to 10.2, and still more preferably within the range of 9.9 to 10.1.

### BRIEF DESCRIPTION OF THE FIGURES

Figures 1 and 2 show two preferred embodiments of the multi-chamber biocompatible plastic bag of the present invention.

### EXAMPLES

The present invention is also illustrated by the following examples, which do not however limit its scope in any way.

### EXAMPLE 1:

A solution of itraconazole with the formula indicated below was prepared:

| | |
|---|---|
| ITRACONAZOLE | 10 mg |
| HYDROXYPROPYL-β-CYCLODEXTRIN | 400.00 mg |
| PROPYLENE GLYCOL | 25.95 mg |
| HCl 37% by weight | 12.12 mg |
| NaOH | q.s. pH 4.00-5.00 |
| WATER FOR INJECTION | q.s. 1 mL |

Hydroxypropyl-β-cyclodextrin was dispersed in water for injection and the resulting dispersion was stirred until complete dissolution. Then, propylene glycol, hydrochloric acid and itraconazole were added to the previous solution. Stirring was continued until a solution was obtained. The pH of this solution was adjusted to pH 4.5 by adding NaOH. Finally, water for injection was added until the final volume. This resulted in a clear, colourless (or pale-yellow), particle-free solution.

### Preparation of a 0.9% NaCl solution (weight/volume)

A solution of NaCl with the formula indicated below was prepared:

| | |
|---|---|
| NaCl | 9 mg |
| 0.5 N HCl or NaOH | q.s. pH 4.5-6.5 |
| WATER FOR INJECTION | q.s. 1 mL |

A solution of the NaCl in water for injection was prepared. The pH of this soution was adjusted to 5.1. Finally, water of injection was added until the desired volume.

### Example 1.A:

### Filling of the double-chamber (chamber A and chamber B) polyolefin bag

The two solutions prepared above were filtered separately using a vacuum pump and 0.22 µm filters.

Using a semi-automatic bag-dosing machine, 25 mL of the 10 mg/mL itraconazole solution was first dosed into chamber A, with a volume of 25 mL, which was then sealed with a port. Then, 50 mL of the 0.9% NaCl solution (weight/volume) was then dosed into chamber B, with a volume of 50 mL, which was also sealed.

The double-chamber bag was subsequently sterilised in an autoclave at 121°C for 15 minuntes to produce a sterile product suitable for injection. The double-chamber bag was covered with a metallised polyester/polyethylene blister with a thickness of 112 µm.

### Example 1.B:

### Filling of the double-chamber (chamber A and chamber B) polyolefin bag

The two solutions prepared above were filtered separately using a vacuum pump and 0.22 µm filters.

Using a semi-automatic bag-dosing machine, 25 mL of the 10 mg/mL itraconazole solution was first dosed into chamber A, with a volume of 26 mL, which was then sealed with a port. Then, 50 mL of the 0.9% NaCl solution (weight/volume) was then dosed into chamber B, with a volume of 51 mL, which was also sealed.

The double-chamber bag was subsequently sterilised in an autoclave at 121°C for 15 minutes to produce a sterile product suitable for injection.

The double-chamber bag was covered with a metallised polyester/polyethylene blister with a thickness of 112 µm.

### EXAMPLE 2:

Double-chamber polyolefin bags (chamber A and chamber B) were prepared as described in example 1 by:
- dosing 20 mL of 10 mg/mL itraconazole solution into chamber A and 40 mL of 0.9% NaCl solution (weight/volume) into chamber B.
- dosing 21 mL of 10 mg/mL itraconazole solution into chamber A and 42 mL of 0.9% NaCl solution (weight/volume) into chamber B.
- dosing 22 mL of 10 mg/mL itraconazole solution into chamber A and 44 mL of 0.9% NaCl solution (weight/volume) into chamber B.
- dosing 23 mL of 10 mg/mL itraconazole solution into chamber A and 46 mL of 0.9% NaCl solution (weight/volume) into chamber B.
- dosing 24 mL of 10 mg/mL itraconazole solution into chamber A and 48 mL of 0.9% NaCl solution (weight/volume) into chamber B.
- dosing 26 mL of 10 mg/mL itraconazole solution into chamber A and 52 mL of 0.9% NaCl solution (weight/volume) into chamber B.
- dosing 27 mL of 10 mg/mL itraconazole solution into chamber A and 54 mL of 0.9% NaCl solution (weight/volume) into chamber B.
- dosing 28 mL of 10 mg/mL itraconazole solution into chamber A and 56 mL of 0.9% NaCl solution (weight/volume) into chamber B.
- dosing 29 mL of 10 mg/mL itraconazole solution into chamber A and 58 mL of 0.9% NaCl solution (weight/volume) into chamber B.
- dosing 30 mL of 10 mg/mL itraconazole solution into chamber A and 60 mL of 0.9% NaCl solution (weight/volume) into chamber B.

### EXAMPLE 3:

A solution of itraconazole with the formula indicated below was prepared:

| | |
|---|---|
| ITRACONAZOLE | 10 mg |
| HYDROXYPROPYL-β-CYCLODEXTRIN | 400.00 mg |
| PROPYLENE GLYCOL | 25.95 mg |
| HCl 37% by weight | 12.12 mg |
| NaOH | q.s. pH 4.00-5.00 |
| WATER FOR INJECTION | q.s. 1 mL |

Hydrochloric acid and propylene glycol were added to water for injection.

A solid dispersion of hydroxypropyl-β-cyclodextrin and itraconazole was prepared separately using the serial dilution process. Once the homogeneity of this solid dispersion was verified, it was added to the hydrochloric acid and the propylene glycol solution and stirred until complete dissolution. The pH of the solution was adjusted to about pH 4.5 by adding the sodium hydroxide solution, and finally, water for injection was added until the desired volume. This resulted in a clear, colourless (or pale-yellow), particle-free solution.

### EXAMPLE 4:

A solution of itraconazole with the formula indicated below was prepared:

| | |
|---|---|
| ITRACONAZOLE | 10.00 mg |
| HYDROXYPROPYL-β-CYCLODEXTRIN | 200.00 mg |
| PROPYLENE GLYCOL | 25.95 mg |
| HCl 37% by weight | 12.12 mg |
| NaOH | q.s. pH 4.00-5.00 |
| WATER FOR INJECTION | q.s. 1 mL |

Itraconazole, hydroxypropyl-β-cyclodextrin, propylene glycol and hydrochloric acid were stirred in water for injection. Once an homogeneous solution of the active ingredient was obtained, the pH was adjusted to about 4.5 by adding the sodium hydroxide solution and finally, water for injection was added until the desired volume. This resulted in a clear, colourless (or pale-yellow), particle-free solution.

### EXAMPLE 5:

A solution of itraconazole with the formula indicated below was prepared as described in example 4:

| | |
|---|---|
| ITRACONAZOLE | 10.00 mg |
| HYDROXYPROPYL-β-CYCLODEXTRIN | 300.00 mg |
| PROPYLENE GLYCOL | 25.95 mg |
| HCl 37% by weight | 12.12 mg |
| NaOH | q.s. pH 4.00-5.00 |
| WATER FOR INJECTION | q.s. 1 mL |

### EXAMPLE 6:

A solution of itraconazole with the formula indicated below was prepared:

| | |
|---|---|
| ITRACONAZOLE | 10.00 mg |
| POLOXAMER 188 | 60.00 mg |
| PROPYLENE GLYCOL | 25.95 mg |
| HCl 37% by weight | 12.12 mg |
| NaOH | q.s. pH 4.00-5.00 |
| WATER FOR INJECTION | q.s. 1 mL |

Itraconazole, poloxamer, propylene glycol and hydrochloric acid were stirred in water for injection. Once an homogeneous solution of the active ingredient was obtained, the pH was adjusted to about 4.5 by adding the sodium hydroxide solution and finally, water for injection was added until the desired volume. This resulted in a clear, colourless (or pale-yellow), particle-free solution.

### EXAMPLE 7:

A solution of itraconazole with the formula indicated below was prepared as described in example 3:

| | |
|---|---|
| ITRACONAZOLE | 8 mg |
| HYDROXYPROPYL-β-CYCLODEXTRIN | 400.00 mg |
| PROPYLENE GLYCOL | 25.95 mg |
| HCl 37% by weight | 12.12 mg |
| NaOH | q.s. pH 4.00-5.00 |
| WATER FOR INJECTION | q.s. 1 mL |

### EXAMPLE 8:

A solution of itraconazole with the formula indicated below was prepared as described in example 3:

| | |
|---|---|
| ITRACONAZOLE | 12 mg |
| HYDROXYPROPYL-β-CYCLODEXTRIN | 400.00 mg |
| PROPYLENE GLYCOL | 25.95 mg |
| HCl 37% by weight | 12.12 mg |
| NaOH | q.s. pH 4.00-5.00 |
| WATER FOR INJECTION | q.s. 1 mL |

### EXAMPLE 9:

The solutions prepared in examples 3-8 were used to fill the chambers of the double-chamber polyolefin bag of the present invention (chamber A and chamber B) as described in examples 1 and 2.

### EXAMPLE 10:

The table below shows the stability results (25°C and 60%RH; 40°C and 75%RH) for the itraconazol solution (10 mg/mL) contained in the chamber A of the double-chamber biocompatible plastic bag of the present invention and the stability results (25°C and 60%RH; 40°C and 75%RH) for the itraconazol solution (10 mg/mL) contained in the type I siliconised glass ampoule of the SPORANOX kit.

| **Itraconazol solution (10 mg/mL) contained in the chamber A of the double-chamber biocompatible plastic bag of the present invention** | | | |
|---|---|---|---|
| | **Time: 0** | **25°C and 60%RH** | **40°C and 75%RH** |
| | | **Time: 6 months** | **Time: 6 months** |
| **Aspect** | Clear solution free of visible particles | Clear solution free of visible particles | Clear solution free of visible particles |
| **Colour** | Less than Y6 | Less than Y6 | Less than Y6 |
| **pH** | 4.6 | 4.6 | 4.6 |
| **EurPh Related substances:** | | | |
| **Impurity B** | 0.10 | 0.10 | 0.10 |
| **Impurity C + D** | <0.05 | <0.05 | <0.05 |
| **Impurity E** | <0.05 | <0.05 | <0.05 |
| **Impurity G** | <0.05 | <0.05 | <0.05 |
| **Individual** | <0.05 | <0.05 | <0.05 |
| **Total** | 0.10 | 0.10 | 0.10 |

| **Itraconazol solution (10 mg/mL) in SPORANOX kit** | | | |
|---|---|---|---|
| | **Time: 0** | **25°C and 60%RH** | **40°C and 75%RH** |
| | | **Time: 6 months** | **Time: 6 months** |
| **Aspect** | Clear solution free of visible particles | Clear solution free of visible particles | Clear solution free of visible particles |
| **Colour** | Less than Y6 | Less than Y6 | Less than Y6 |
| **pH** | 4.5 | 4.6 | 4.5 |
| **EurPh Related substances:** | | | |
| **Impurity B** | <0.05 | <0.05 | <0.05 |
| **Impurity C + D** | 0.15 | 0.13 | 0.12 |
| **Impurity E** | <0.05 | <0.05 | <0.05 |
| **Impurity G** | 0.06 | 0.05 | 0.05 |
| **Individual** | <0.05 | <0.05 | <0.05 |
| **Total** | 0.21 | 0.18 | 0.17 |

## Claims

1. A kit comprising a multi-chamber container **characterised in that** the multi-chamber container comprises at least one chamber A and at least one chamber B, wherein the chamber A contains a pharmaceutically acceptable solution of at least one active ingredient and the chamber B contains a pharmaceutically acceptable solution for the reconstitution of the solution contained in the chamber A.

2. A kit according to claim 1 **characterised in that** the multi-chamber container is a container comprising one chamber A and one chamber B, wherein the chamber A contains a pharmaceutically acceptable solution of at least one active ingredient and the chamber B contains a pharmaceutically acceptable solution for the reconstitution of the solution contained in the chamber A, so that the container contains the means for mixing the solution contained in the chamber A and the solution contained in the chamber B inside the same multi-chamber container by means of total or partial rupture of the barrier between the chamber A and the chamber B.

3. A kit according to claim 2 **characterised in that** the multi-chamber container is a multi-chamber biocompatible plastic bag, preferably a double-chamber biocompatible plastic bag.

4. A kit according to any one of the precceding claims **characterised in that** the chamber A contains a pharmaceutically acceptable solution, preferably a pharmaceutically acceptable aqueous solution, of at least one poorly water-soluble active ingredient.

5. A kit according to claim 4 **characterised in that** the poorly water-soluble active ingredient is an antifungal agent, wherein the antifungal agent is an azole derivative, preferably itraconazole, fluconazole, voriconazole, posaconazole, clotrimazole, ketoconazole, econazole, saperconazole, ravuconazole, isavuconazole, miconazole or tioconazole, more preferably itraconazole.

6. A kit according to claim 5 **characterised in that** the pharmaceutically acceptable solution contained in the chamber A is a pharmaceutically acceptable aqueous solution of the azole derivative, preferably itraconazole, with a concentration comprised of from 1 mg/mL to 30 mg/mL, preferably comprised of from 5 mg/mL to 20 mg/mL, and more preferably about 10 mg/mL.

7. A kit according to any one of claims 5 or 6 **characterised in that** the pharmaceutically acceptable solution contained in the chamber A comprises at least one inclusion complex of the azole derivative-cyclodextrin, preferably itraconazole-cyclodextrin, wherein the cyclodextrin is selected from the group consisting of α-, β- and γ-cyclodextrins optionally substituted by hydroxyethyl, hydroxypropyl, dihydroxypropyl, methylhydroxyethyl, methylhydroxypropyl, ethylhydroxyethyl, ethylhydroxypropyl, glucosyl, maltosyl, maltotriosyl or sulfoalkly ether groups, preferably hydroxypropyl-β-cyclodextrin.

8. A kit according to any one of claims 4 to 7 **characterised in that** the pharmaceutically acceptable solution contained in the chamber A further comprises a polymeric solubiliser or a mixture thereof, preferably a copolymer of polyoxyethylene-polyoxypropylene, more preferably poloxamer 188.

9. A kit according to any one of claims 4 to 8 **characterised in that** the pharmaceutically acceptable solution contained in the chamber A further comprises one or more co-solvents selected from the group consisting of ethanol, propylene glycol, oils and glycerine, preferably propylene glycol.

10. A kit according to any one of claims 4 to 9 **characterised in that** the pharmaceutically acceptable solution contained in the chamber A further comprises a pharmaceutically acceptable acid, preferably hydrochloric acid.

11. A kit comprising a double-chamber biocompatible plastic bag comprising one chamber A and one chamber B, **characterised in that** the chamber A contains a volume comprised of from 20 mL to 30 mL of a pharmaceutically acceptable aqueous solution of itraconazole with a concentration about 10 mg/mL and the chamber B contains a volume comprised of from 40 mL to 60 mL of a pharmaceutically acceptable NaCl solution with a concentration about 0.9% (weight/volume).

12. A kit according to claim 11 charaterized in that the solution contained in the chamber A further comprises hydroxypropyl-β-cyclodextrin, propylene glycol and hydrochloric acid.

13. A kit according to any one of claims 11 or 12, **characterised in that** the chamber A contains about 25 mL of the aqueous itraconazole solution with a concentration about 10 mg/mL and the chamber B contains about 50 mL of the NaCl solution with a concentration about 0.9% (weight/volume).

14. A kit according to any one of the precceding claims which further comprises a blister, preferably metallised, which covers the multi-chamber container and an in-line filter and, optionally, an extension line consisting of a plastic tube with a two-way shut-off valve.

15. A kit according to any one of the precceding claims for use as a medicinal product.
